# EUROPEAN PATENT APPLICATION

(11) **EP 4 684 805 A1**
(43) Date of publication of application: **28.01.2026**
(21) Application number: 24190636.1
(22) Date of filing: 24.07.2024
(51) Int. Cl.: A61K 47/68, A61P 35/00

(54) **SELECTIVE ANTIBODY-DRUG CONJUGATES FOR TARGETED CANCER THERAPY**

(71) Applicant: Universitätsmedizin der Johannes Gutenberg-Universität Mainz, 55131 Mainz (DE); Johannes Gutenberg-Universität Mainz, 55122 Mainz (DE)
(72) Inventor: BOPP, Tobias, 65187 Wiesbaden (DE); BESENIUS, Pol, 55128 Mainz (DE); ATTARIYA-HEFNAWY, Riem, 55130 Mainz (DE); STERGIOU, Natascha, 1051VC Amsterdam (NL); KUNZ, Horst, 55127 Mainz (DE); SCHMITT, Edgar, 55127 Mainz (DE)
(74) Representative: Patentanwälte Dr. Keller, Schwertfeger Partnerschaft mbB

(57) **Abstract**

The invention relates to antibody drug conjugates comprising an antibody that specifically binds to tumor-associated Mucin 1 (TA-MUC1) of human epithelial tumor cells, but not to highly glycosylated MUC1 on healthy epithelial cells, wherein the antibody is coupled to a cytotoxic agent selected from the group consisting of a tubulin inhibitor or a topoisomerase inhibitor. The invention furthermore relates to a pharmaceutical composition, comprising such an antibody drug conjugate for use in the treatment of malignant epithelial tumors.

## Description

### Field of the Invention

The present invention relates to the field of targeted cancer therapeutics, specifically antibody-drug conjugates that selectively target tumor-associated antigens for the treatment of malignant epithelial tumors.

### Background of the Invention

Cancer remains one of the most significant health challenges globally, affecting millions each year with a wide range of malignancies that vary in behavior, treatment response, and prognosis. According to the World Health Organization, cancer is the second leading cause of death worldwide, with approximately 10 million deaths in 2020 alone. The incidence of cancer is expected to rise to over 29 million new cases annually by 2040 due to the aging and growth of the global population, alongside increasing prevalence of risk factors such as smoking, diet, and physical inactivity.

Antibody-drug conjugates (ADCs) represent a significant advancement in the field of oncology, offering a targeted approach to cancer therapy that combines the specificity of monoclonal antibodies with the potent cytotoxic effects of traditional chemotherapy agents. This hybrid technology leverages the unique properties of antibodies to deliver toxic drugs directly to cancer cells, minimizing damage to healthy tissues and improving the efficacy of cancer treatment.

An ADC usually comprises a monoclonal antibody, which is designed to specifically bind to an antigen that is selectively expressed on the surface of cancer cells and a cytotoxic drug, which is typically a potent anti-cancer agent that can kill cells by interfering with critical cellular processes such as DNA replication or microtubule function. The antibody is usually linked to the cytotoxic drug via a linker. The ADC circulates through the body until it encounters and binds to the tumor-specific target antigen on the surface of a cancer cell. After binding to the antigen, the ADC-antigen complex is internalized by the cancer cell through endocytosis. Once inside the cell, the linker is cleaved, releasing the cytotoxic drug. The released drug induces cell death by its mechanism of action, which typically involves disrupting critical processes such as DNA synthesis or spindle formation during cell division.

Malignant epithelial tumors such as breast, pancreatic, lung, colon, prostate, bladder cancers, squamous cell carcinoma, and endometrial adenocarcinoma, frequently express tumor-associated Mucin 1 (TA-MUC1), which is absent in healthy tissues. Unlike normal MUC1, which is present on healthy epithelial cells but typically highly glycosylated, TA-MUC1 exhibits an aberrant glycosylation pattern. This alteration not only differentiates cancer cells from non-cancerous cells but also affects the molecule's physical properties and biological behavior. TA-MUC1 benefits the tumor in several significant ways that contribute to the progression and complexity of cancer, particularly breast cancer. TA-MUC1 is overexpressed in more than 90% of all breast tumors.

Antibodies that recognize TA-MUC1 and its characteristic glycosylation pattern in the context of cancer therapy have been described, for instance, in WO 2019/179923 A1. The monoclonal antibody GGSK-1/30 that recognizes human TA-MUC1 has been described as a screening tool in the context of diagnosis and prognosis of breast cancer (Stergiou N, Nagel J, Pektor S, Heimes AS, Jäkel J, Brenner W, Schmidt M, Miederer M, Kunz H, Roesch F, Schmitt E. Evaluation of a novel monoclonal antibody against tumor-associated MUC1 for diagnosis and prognosis of breast cancer. Int J Med Sci. 2019 Aug 14;16(9):1188-1198). The tumor selectivity of monoclonal antibody GGSK1/30 is particularly high and versatile, and not only directed against breast tumors, but also against other epithelial tumors, e. g. pancreas tumor tissues (Angew, Chem Int. Ed. 2016, 55, 2894-2898).This is because the antibody is induced not by a proteolytic mixture obtained from tumor cells, but by a structurally exactly specified, synthetic glycopeptide antigen representing a partial structure of tumor-associated MUC1 linked through an immunologically inert spacer to a T-cell stimulating component, in particular, tetanus toxoid (N. Gaidzig et al. Angew, Chem Int. Ed. 2011, 50, 9977-9981). Its tumor selectivity is also demonstrated by the fact that its binding to the tumor cells can by completely blocked by addition of the synthetic glycopeptide antigen.

Although antibody therapies and potential targets are known, there is still an unmet medical need to increase efficacy and selectivity in antibody-related treatments in particular with respect to malignant epithelial tumor diseases. It would therefore be desirable to increase efficacy and selectivity of known TA-MUC1-specific antibodies such that they exhibit improved properties in cancer therapy.

### Summary of the Invention

Against this background, it is the object of the present invention to provide improved antibody-based drugs based on a TA-MUC1 antibody for use in the treatment of malignant epithelial tumors.

The solution of this problem is provided by the antibody drug conjugate as defined in the claims. Advantageous embodiments of the invention can be found in the dependent claims.

It will be apparent that the invention can be further improved or further characterized by the features described herein or advantageous embodiments, each of which is advantageous in itself and can be combined with one another as desired.

In a first aspect, the invention relates to an antibody drug conjugate (ADC) comprising an antibody that selectively binds to tumor-associated Mucin 1 (TA-MUC1) of human epithelial tumor cells, but not to highly glycosylated healthy epithelial cells. According to the present invention, the TA-MUC1-specific antibody is coupled to a cytotoxic agent selected from the group consisting of a tubulin inhibitor or a topoisomerase inhibitor.

The ADCs of the invention are in particular advantageous because they are characterized by high tumor selectivity and affinity. These properties make the inventive ADCs excellent candidates for the treatment of tumor diseases that were previously insufficiently treatable, in particular malignant epithelial tumors.

A tubulin inhibitor as used in the ADC of the present invention relates to a cytotoxic agent that interferes with the function of tubulin, a protein that is a key component of the microtubule structure within cells. Microtubules are critical for a variety of cellular processes, including maintaining the structure of the cell, intracellular transport, and most importantly, cell division. By targeting tubulin, the tubulin inhibitor disrupts microtubule dynamics, which can halt cell division, leading to cell death. This mechanism is particularly effective against rapidly dividing cells, such as cancer cells.

In some embodiments, the tubulin inhibitor that is coupled to the TA-MUC1-specific antibody is a taxane drug, preferably selected from paclitaxel, docetaxel or vinorelbine.

A topoisomerase inhibitor as used in the ADC of the present invention relates to a class of drugs that interfere with the action of topoisomerase enzymes that leads to breaks in the DNA, which can trigger cell death, particularly in rapidly dividing cells such as cancer cells.

In some embodiments, the topoisomerase inhibitor is a topoisomerase I inhibitor selected from the group consisting of irinotecan (CPT-11) and topotecan. These drugs stabilize the transient break caused by topoisomerase I in the single DNA strand, preventing re-ligation and leading to single-strand breaks.

In some other embodiments, the topoisomerase inhibitor is a topoisomerase II inhibitor selected from the group consisting of etoposide (VP-16), doxorubicin or mitoxantrone.

In particularly advantageous embodiments, the invention relates to ADCs comprising tubulin and topoisomerase inhibitors that have been proven to effectively target epithelial tumor cells expressing TA-MUC1.

In the course of developing the invention, three candidates of tubulin and topoisomerase inhibitors were found as examples for cytotoxic agents that exhibit great therapeutic potential in the treatment of malignant epithelial tumor diseases such as breast cancer or pancreas cancer when coupled to TA-MUC1-specific antibodies. These highly efficient tubulin and topoisomerase inhibitors are coupled to an antibody, preferably a monoclonal antibody, that recognizes and specifically binds to TA-MUC1 in order to generate the ADCs of the invention.

In one aspect, the present invention therefore relates to an antibody drug conjugate comprising an antibody that specifically binds to TA-MUC1 of human epithelial tumor cells, but not to MUC1 on highly glycosylated healthy epithelial cells, wherein the antibody is coupled to a tubulin inhibitor, which is emtansine (DM1).

Emtansine, often referred to by its abbreviation DM1, is a derivative of maytansine and is considered to be a potent cytotoxic agent for cancer therapy. As a component of ADCs, DM1 provides effective anti-cancer activity with reduced systemic toxicity when coupled to TA-MUC1 antibodies.

DM1 inhibits microtubule assembly by binding to tubulin at the rhizoxin binding site, which is near the vinca alkaloid binding site but distinct in its interaction. By interfering with microtubule dynamics, DM1 effectively blocks cell division at the mitotic phase, inducing apoptosis in rapidly dividing cancer cells. This mechanism is particularly useful in targeting the cancer cells while sparing normal cells when delivered specifically through the antibody component of the ADC. The targeted delivery mechanism of ADCs ensures a high accumulation of DM1 in the tumor tissue that would not be feasible with systemic administration.

In some embodiments, DM1 is covalently linked to antibodies through a stable linker that can be cleaved inside cancer cells, releasing the cytotoxic agent where it is needed most. This minimizes the exposure of non-cancerous tissues to the drug, thereby reducing side effects associated with traditional chemotherapy.

In another aspect, the present invention relates to an antibody drug conjugate comprising an antibody that specifically binds to tumor-associated Mucin 1 (TA-MUC1) of human epithelial tumor cells, but not to MUC1 on highly glycosylated healthy epithelial cells, wherein the antibody is coupled to a topoisomerase inhibitor, which is deruxtecan (DX) or SN-38.

DX and SN-38 are both topoisomerase I inhibitors, each having distinct characteristics and roles in cancer treatment. SN-38 is the active metabolite of the prodrug irinotecan (CPT-11), a well-known chemotherapy medication used primarily in the treatment of colorectal cancer. SN-38 is significantly more potent than its precursor and functions by inhibiting Topoisomerase I. In particular, SN-38 prevents the re-ligation of the single-strand breaks made by the topoisomerase enzyme, resulting in the accumulation of DNA damage, which eventually leads to cell death, particularly in rapidly dividing tumor cells. DX is similar in function to SN-38, inhibiting Topoisomerase I and inducing substantial DNA damage leading to cell death.

All three candidates have been tested in their potential to delay or stop tumor progression. In order to evaluate the therapeutic potential of the ADCs equipped with DM1, DX or SN-38, mice bearing a palpable mammary tumor were treated with the three different ADCs. As shown by the present invention, tumor progression was halted in all three cases after intraperitoneal injection within a few days, and in the following two to three weeks the tumor was eradicated in all animals. The tumoricidal effect of the immunotherapeutics used was based on the binding of ADC to expressed human TA-MUC1 in comparison to control experiments using a mouse breast tumor line that did not express human MUC1.

In parallel to these studies, biopsies from a number of different tumors were analyzed immunohistochemically using the antibody of the present invention, preferably the GGSK-1/30 monoclonal antibody. This showed that various human epithelial tumor entities and their metastases were selectively detected, including pancreatic tumors, which are known to be particularly aggressive and dangerous. The ADCs of the present invention are therefore suitable drugs in the treatment of pancreatic tumors and various other epithelial tumor entities.

In the case of ADCs comprising the cytotoxic agent DX, the conjugate allows for the precise delivery of the cytotoxic agent to cancer cells that express specific antigens, enhancing the drug's efficacy and reducing collateral damage to healthy cells. On the other side, the cytotoxic agent SN-38 is extremely potent, allowing lower doses to be used for effective therapy, which can help in reducing the side effects compared to other chemotherapeutic agents.

As shown herein, the tubulin inhibitor DM1 and the topoisomerase inhibitor DX or SN-38 are linked to the antibody by one or more linking amino acids that form a linker. Preferably the linker is selected from the group consisting of cleavable linkers, non-cleavable linkers, peptide-based linkers or hydrazone linkers. Cleavable linkers are designed to be stable in the bloodstream but can be cleaved in the tumor environment by specific conditions or enzymes. For example, they can be cleaved by the low pH within lysosomes or by specific proteases that are abundant in tumor tissues. Non-cleavable linkers are designed not to be cleaved. This means that the entire ADC molecule, including linker and cytotoxic agent, is internalized by the target cell. The antibody part of the ADC is then degraded by lysosomal enzymes, releasing the cytotoxic agent while being still attached to the linker. Peptide-based linkers contain a peptide sequence that can be cleaved by lysosomal proteases once the ADC is internalized by the target cell. Hydrazone linkers are pH-sensitive linkers that are stable at the neutral pH of the bloodstream but hydrolyze in the acidic environment of cell lysosomes.

In some embodiments, the cytotoxic agents can be linked to the antibody of the ADC through a lysine (-NH₂) or cysteine residue (-SH). When linked to the antibody via cysteine residues, after reducing the interchain disulfide bonds in antibodies, new thiol groups are exposed on cysteine residues, which can be used for conjugation. This strategy typically results in a more uniform ADC product, as the number of interchain disulfide bonds (hence the cysteine residues available for conjugation) is limited and more predictable. The specific design and type of linker used are critical in determining the therapeutic window, efficacy, and safety profile of ADCs like those incorporating deruxtecan. The precision with which these components are selected and engineered determines the success of the ADC in clinical applications, balancing potent antitumor activity with minimal side effects.

In some embodiments, DM1 is linked via succinimidyl-trans-4-(maleimidylmethyl) cyclohexane-1-carboxylate (SMCC) to a lysine residue (-NH₂) of the antibody. SMCC is a stable thioether linker that binds primarily to antibody lysine residues. In alternative embodiments, a maleimidocaproyl linker (MC linker) is used, which is a non-cleavable linker which will be coupled through the cysteine residue of the antibody. The MC molecule can also be covalently connected via a peptide spacer comprising the amino acids GGFG (Gly Gly Phe Gly), which is assumed to be stable in circulation, and is cleaved by lysosomal enzymes.

In some embodiments, the linker of the ADC is designed to react with the amino groups of lysine residues in the antibody. This strategy can result in a heterogeneous mixture of ADC molecules, as the antibody contains multiple lysine residues that can be conjugated with the cytotoxic agent such as DM1.

In one aspect, the cytotoxic agent coupled to the antibody via the lysine residue is SMCC-DM1 comprising the formula: 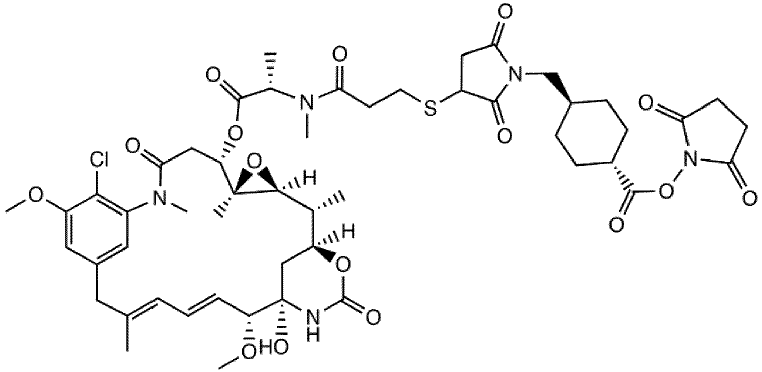 wherein SMCC is the linker succinimidyl-trans-4-(maleimidylmethyl) cyclohexane-1-carboxylate and DM1 is the cytotoxic agent emtansine.

In a further aspect, the cytotoxic agent coupled to the antibody is deruxtecan (DX) linked via maleimidocaproyl (MC) which is covalently connected via a Gly Gly Phe Gly peptidyl spacer (GGFG) to a cysteine residue (-SH) of the antibody. The linker used in the antibody-DX construct is preferably a cleavable linker, which is designed to be stable during circulation in the bloodstream but to be cleaved once inside the target cancer cell. This selective activation is crucial for minimizing the systemic toxicity of the cytotoxic drug while maximizing its efficacy at the tumor site.

For deruxtecan (DX), the conjugation to the antibody preferably is done via cysteine residues involving a process that comprises the partial reduction of interchain disulfide bonds in the antibody. This process exposes thiol groups that can be chemically modified to attach the linker-drug complex:
Cysteine conjugation in this case is preferred because it tends to provide a more uniform drug-to-antibody ratio (DAR), which is crucial for the consistent performance of the ADC. The partial reduction of disulfide bonds allows for the introduction of the linker-drug complex to cysteine residues, forming a stable thioether bond.

In another preferred embodiment, the topoisomerase inhibitor of the ADC is SN-38 linked via maleimidocaproyl (MC) to a cysteine residue (-SH) of the antibody.

In an even more preferred embodiment, the cytotoxic agent coupled to the antibody via the cysteine residue is MC-GGFG-DX comprising the formula: 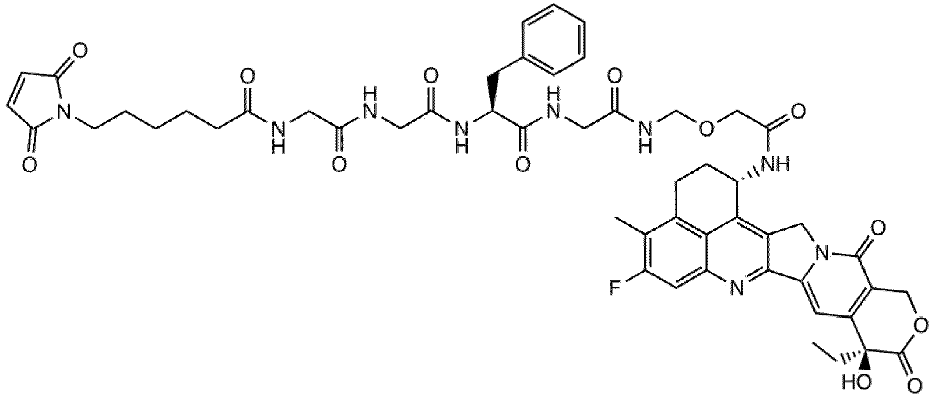 wherein MC is the linker maleimidocaproyl, GGFG is a peptidyl spacer und DX is the cytotoxic agent deruxtecan.

In a preferred embodiment, the cytotoxic agent coupled to the antibody via the cysteine residue is MC-SN-38 comprising the formula: 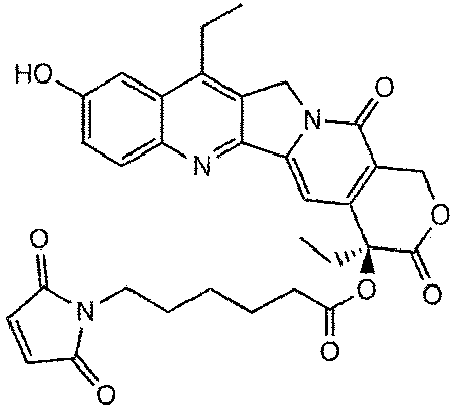 wherein MC is the linker maleimidocaproyl and SN-38 is the cytotoxic agent.

In the novel three ADCs that have been used as examples to illustrate the scope of the invention, the tubulin inhibitor DM1 is coupled via a SMCC linker to -NH₂ of lysine, DX is coupled via maleimide-GGFG peptide linker to -SH of cysteine, and SN-38 is coupled via maleimide linker of -SH of cysteine.

It is apparent that the antibody component of the ADC can be any one that specifically recognizes TA-MUC1, including, but not limited to polyclonal or monoclonal antibodies. In a preferred embodiment, the antibody of an ADC is a monoclonal antibody directed against TA-MUC1, preferably human TA-MUC1 (hu-TA-MUC1). In a particularly preferred embodiment, the monoclonal antibody is GGSK-1/30.

In some embodiments, the antibody component of the ADC comprises the TA-MUC1 binding sites of the amino acid sequences of SEQ ID NO: 1 to SEQ ID NO: 6.

In more particular, the GGSK-1/30 antibody component of ADC of the present invention comprises two polypeptides bearing the TA-MUC1-specific binding sites (CDR1-3 of heavy and light chain), namely
- a first polypeptide sequence of the V-H chain comprising three different amino acid sequences selected from the group consisting of GFTFSDYW (SEQ ID NO: 1), IRLKSNNYAA (SEQ ID NO: 2) and TFGNSFAY (SEQ ID NO: 3),
- a second polypeptide sequence of the V-L chain comprising three different amino acid sequences selected from the group consisting of TGAVTTNNY (SEQ ID NO: 4), GTN (SEQ ID NO: 5) and ALWYSNHWV (SEQ ID NO: 6),

In some embodiments, the antibody is a monoclonal antibody comprising the amino acid sequence of any one of SEQ ID NO: 7 to 9.

In a further aspect, the invention relates to an antibody drug conjugate (ADC) of the monoclonal antibody GGSK-1/30 for use in the treatment of malignant epithelial tumors. An epithelial tumor, also known as an epithelial carcinoma or carcinoma, is a type of cancer that originates from epithelial cells. In a preferred embodiment, the malignant epithelial tumor is selected from the group consisting of breast cancer, pancreatic cancer, lung cancer, colon cancer, prostate cancer, bladder cancer, squamous cell carcinoma, endometrial adenocarcinoma.

In a further aspect, the present invention relates to a pharmaceutical composition, comprising an antibody drug conjugate (ADC) as described herein. Such compositions typically comprise the ADC as disclosed herein and a pharmaceutically acceptable carrier. As used herein, the term "pharmaceutically acceptable carrier" has its ordinary and customary meaning as read in light of this disclosure, and may include any solvents, dispersion media, coatings, antibacterial and antifungal agents, isotonic and absorption delaying agents, and the like, compatible with intended route of administration. Suitable carriers are described in the most recent edition of Remington's Pharmaceutical Sciences, a standard reference text in the field. In some embodiments, examples of such carriers or diluents include, but are not limited to water, saline, ringer's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated for some embodiments. In some embodiments, supplementary active compounds are also incorporated into the pharmaceutical compositions.

In some embodiments, in a pharmaceutical composition or use of any one of the embodiments disclosed herein, a therapeutically effective amount of ADC is used. As used herein, "therapeutically effective amount" or "effective amount" of the ADC of the disclosure has its plain and ordinary meaning as understood by one of skill in the art in view of the present disclosure, and relates generally to the amount needed to achieve a therapeutic objective. In some embodiments, this may be a complete to partial recovery from malignant epithelial tumors of a human subject in need thereof. In some embodiments, this may be a partial or complete prevention of development of a malignant epithelial tumor of a human subject in need thereof.

In some embodiments, the pharmaceutical composition of any one of the embodiments disclosed herein is formulated to be compatible with its intended route of administration as disclosed herein. Examples of routes of administration, some of which are used in embodiments disclosed herein, include parenteral, e.g., intraarticular, intravenous, intradermal, subcutaneous, oral (e.g., inhalation), transdermal (i.e., topical), transmucosal, and rectal administration. Examples of solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose.

In some embodiments, pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In some embodiments, the composition is sterile and should be fluid to the extent that easy syringeability exists. In some embodiments, it is stable under the conditions of manufacture and storage and is preserved against the contaminating action of microorganisms such as bacteria and fungi. In some embodiments, the carrier can be a solvent or dispersion medium containing, for example, water, ethanol, polyol (for example, glycerol, propylene glycol, and liquid polyethylene glycol, and the like), and suitable mixtures thereof.

In some embodiments, the proper fluidity can be maintained, for example, by the use of a coating such as lecithin, by the maintenance of the required particle size in the case of dispersion and by the use of surfactants. In some embodiments, prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

In some embodiments, sterile injectable solutions can be prepared by incorporating the ADC in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In conclusion, the development of an ADC derived from the monoclonal antibody GGSK-1/30 that specifically targets TA-MUC1 offers a promising therapeutic strategy for the treatment of cancers expressing this antigen, with potential for high efficacy, reduced toxicity, and integration into personalized treatment plans. The ADCs of the present invention targeting TA-MUC1 showed inhibition of tumor growth and elimination of the tumor. It is intended that they can be used in combination with other therapeutic agents, such as hormone therapies, immune checkpoint inhibitors, or other targeted therapies, to enhance overall treatment efficacy and potentially overcome resistance to single-agent therapies.

### Short Description of the Figures

Figure 1 A/B show the synthetic route for the preparation of examples of three ADCs.
Figure 2 shows the setup of the in vivo experiments.
Figure 3 demonstrates the effect of the ADCs on tumor growth in a first in vivo experiment.
Figure 4 demonstrates the effect of the ADCs on tumor growth in a second in vivo experiment.
Figure 5 shows the results of in vitro cytotoxicity assays.

### Examples

The present disclosure is further illustrated by the following non-limiting examples, the teachings and disclosure of which can be generalized and combined with the foregoing disclosure.

The examples show that the high tumor selectivity of antibody GGSK1/30 that can be obtained by a method developed by Kunz and Schmitt (Angew, Chem Int. Ed. 2009, 48, 7551-7555; Angew, Chem Int. Ed. 2005, 44, 7630-7635; Angew, Chem Int. Ed. 2001, 40, 366-360) is not affected by the conjugation with cytotoxic drugs in antigen drug conjugates (ADCs), and the generated ADCs of the invention are a valuable tool for antitumor therapy, particularly for the treatment of malignant epithelial tumors.

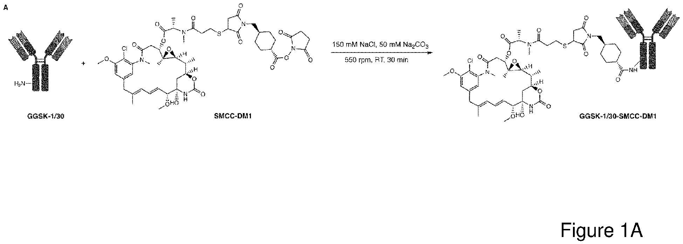

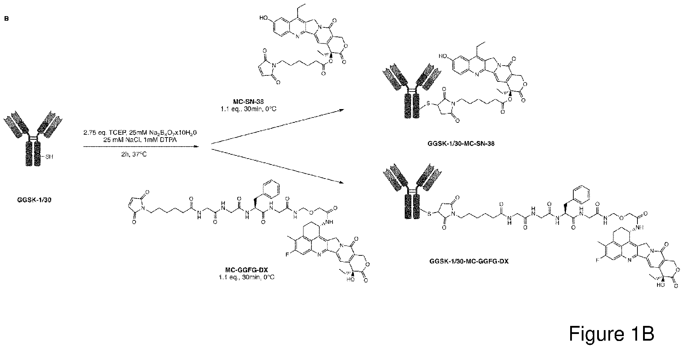

Figures 1 A/B illustrate the synthetic route of three ADCs of the invention (DM1, DX, SN-38). Figure 1A shows the steps for constructing GGSK-1/30-DM1 ADC in basic conditions coupling the cytotoxic agent to the amine side of the GGSK-1/30 antibody using a SMCC linker. Figure 1B shows the steps for constructing GGSK-1/30-SN-38 and GGSK-1/30-DX to couple the cytotoxic agent on to the thiol group of the antibody using a MC and MC-GGFG linker, respectively.

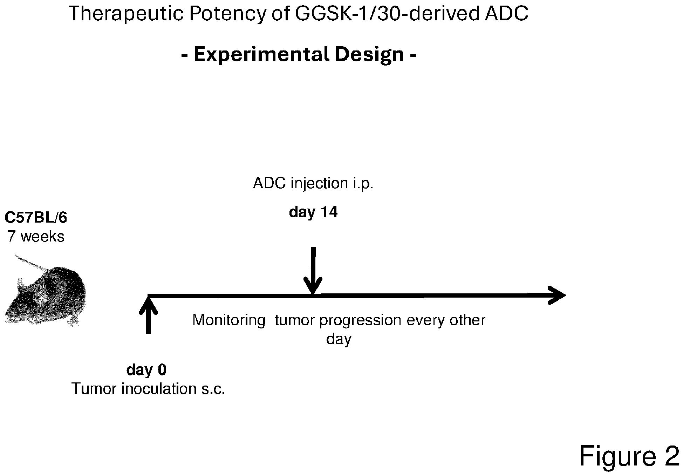

Figure 2 shows the schematic of the in vivo experiments conducted in the course of the present invention. At day 0 mouse tumor cells expressing human MUC1 were injected s.c. into seven weeks old C57BL/6 mice. Subsequently the mice were treated intraperitoneally (i.p.) with 200 µg ADC at day 14. Every other day the tumor growth was determined. As a control, non-treated tumor-bearing mice were used.

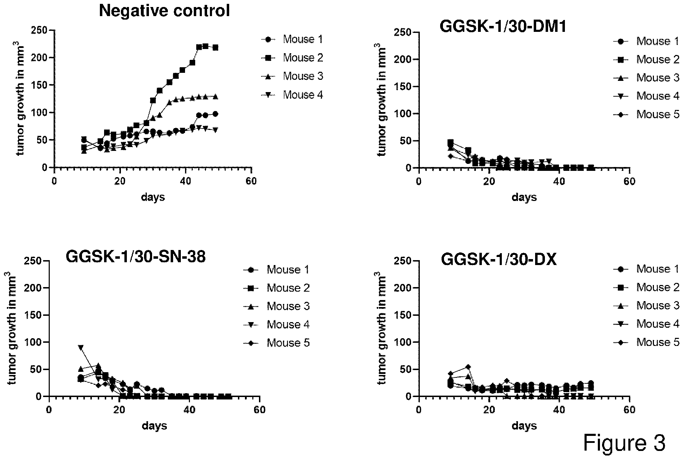

Figure 3 demonstrates the effect of the ADCs on tumor growth in a first in vivo experiment. C57BL/6 mice suffering from solid tumors were treated with 200 µg of the GGSK 1/30 based ADC derivates. Groups of 5 mice were treated with ADCs containing DM1, DX, or SN-38 two weeks after s.c. tumor inoculation. Tumor growth was assessed for each mouse every other day as depicted in the figure.

In order to evaluate the *in vivo* anti-tumor efficacy of the human TA-MUC1 ADC groups of tumor-bearing C57BL/6 mice were treated intraperitoneally with a single injection (200µg/100µl) of three different ADCs. Tumor growth was repetitively monitored every other day starting two weeks after tumor inoculation. Tumor progression in mice which were treated solely with PBS served as negative control. In general, all of the groups treated with ADCs exhibited at least an arrest of tumor growth. The injection of GGSK-1/30-DM1 led to tumor size reduction in all mice of this group followed by complete eradication of the tumor. The same result could be observed in the group that was treated with the GGSK-1/30-SN-38 ADC. Concerning DX-containing ADC treatment tumor progression was arrested in two of the mice while in the remaining three mice tumors were eradicated approximately 50 days after the application of the ADC.

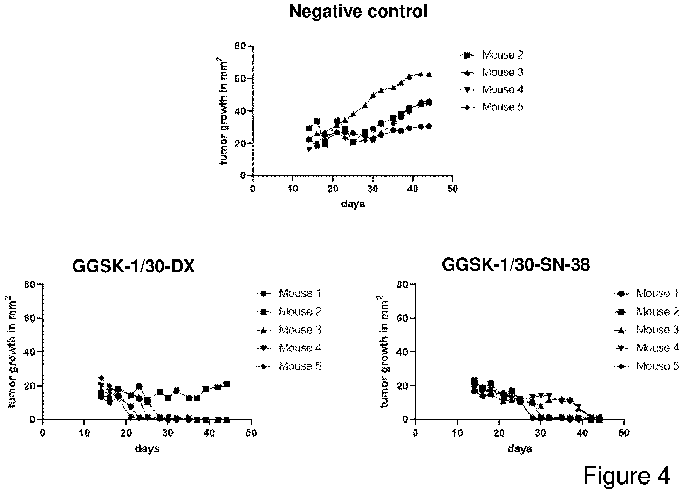

Figure 4 demonstrates the effect of the ADCs on tumor growth in a second in vivo experiment. Groups of five C57BU6 mice suffering from solid tumors were treated with 200 µg/100 µl GGSK-1/30-SN-38 or GGSK-1/30-DX two weeks after tumor cell injection. Non-treated mice served as a negative control. Tumor burden increases continually in the negative control while the tumor load was significantly reduced in ADC-treated mice.

Similar results as compared to the previous experiment (see Figure 3) could be observed. Concerning GGSK-1/30-SN-38 ADC, tumors in all mice were eradicated 44 days after inoculation. The same result could be observed in four mice treated with GGSK-1/30-DX ADC. Only one mouse of this group bared a palpable tumor that was growth-arrested after treatment with GGSK-1/30-DX ADC. These results illustrate the effectiveness of an ADC treatment in cancer therapy.

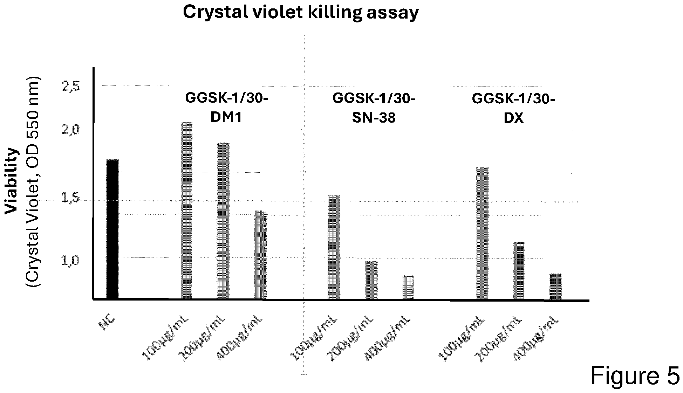

Figure 5 shows the results of the in vitro cytotoxicity assays. When cells are exposed to ADC molecules, antigen-expressing cells can effectively take up those molecules and eventually die as a result of the released cytotoxic payload.

ADCs were co-cultured with human MUC1-expressing mouse PyMT tumor cells. Representative figure of crystal violet staining after co-culture of GGSK-1/30 ADCs with human MUC1-positive PyMT murine tumor cells for 7 days. ADCs containing the three toxins emtansin (DM1), SN-38 and deruxtecan (DX) given in three different concentration including 100 µg/mL, 200µg/mL and 400µg/mL were compared to the negative control (NC).

The cell surface expression of human MUC1 was evaluated by flow cytometry analysis. GGSK-1/30 ADCs showed potent dose-dependent cytotoxicity in vitro against the tumor cells that expressed human MUC1. The ability of the ADCs to induce cell lysis of such tumor cells was investigated in a photometrically based killing assay (Figure 5). To this end, the ADCs were incubated with the tumor cells in different concentrations for 7 days. An indirect quantification of cell death is based on the vital staining of living cells with crystal violet. Mouse PyMT tumor cells that do not express human MUC1 served as a control.

By increasing the concentration of the ADCs, a strong dose-dependent cytolytic activity could be observed especially for the cells incubated with SN-38 and DX. After 7 days of incubation at least 80% of cell lysis was observed in the presence of 400 µg/ml of GGSK-1/30-SN-38 and -DX. The GGSK-1/30-DM1 conjugate showed a comparatively weak effect on tumor cell viability reducing the vital staining to approximately 50%. Tumor cell viability increased in the presence of lower concentrations (200 µg/ml, 100 µg/ml) of the GGSK-1/30 ADCs.

### Material & Methods

### Construction of the ADCs

For the generation of the ADCs, GGSK-1/30-DM1 13,3 nmol of GGSK-1/30 were mixed with 150mM NaCl, 50mM Na₂CO₃ and 53 nmol of DM1-SMCC (nonreducible thioether linker) and incubated for 30 mins at room temperature in a thermomixer at 550 rpm. Reaction with the thiol-containing maytansinoid DM1 proceeded smoothly to give a conjugate with an average of two DM1 molecules linked via thioether bonds. The ADC was purified using a PD-10 column (GE Healthcare).

GGSK-1/30 antibodies were treated with 2.75 molar equivalent of tris(2-carboxyethyl) phosphine hydrochloride (TCEP) in 0.025 M sodium borate pH 8, 0.025 M NaCl, 1 mM DTPA for 2 h at 37°C. Without purification, the mixture was then cooled to 0°C and partially reduced GGSK-1/30 was reacted with 1.1 molar equivalent MC-SN-38 or MC-GGFGDX. The conjugation reaction was performed at 0°C for 30 min. GGSK-1/30-SN-38 and GSK-1/30-DX were purified also using the PD-10 columns, which were equilibrated with PBS.

### Cytotoxicity assay

An assay was established to evaluate the cytotoxic potency of the newly generated GGSK-1/30 ADCs by using mouse PyMT tumor cells that express human MUC1. These target cells (1x 10⁴ /well) were plated in 200 µL culture medium into a 96-well plate and incubated overnight to allow attachment onto the plate. Subsequently, the GGSK-1/30-ADCs (100 µg/mL, 200 µg/mL and 400 µg/mL) were added in 50 µL/well to triplicate cultures for 7 d. To visualize the cytolytic activity of the ADCs, the remaining vital target cells were stained with crystal violet. To this end, the medium was aspirated, and the remaining tumor cells were carefully washed with PBS. Afterwards, cells were fixed by the addition of 300 µL ice-cold methanol-acetone mixture (1:1) and subsequently incubated at 4 °C for 10 min. After this incubation period, the fixation mixture was discarded and 100 µL crystal violet staining solution (0.5 g crystal violet, 80 mL water, 20 mL methanol) was added and incubated for 220 min at RT. The staining solution was discarded, wells were washed with water and 125 µL of the measuring solution (50% µL of the measuring solution (50% ethanol in 0,1% acetic acid)) were added and the absorption measured on the Tecan reader at 550 nm.

### Evaluation of the therapeutic potency of GGSK-1/30 ADCs

C57BL/6 mice were inoculated seven weeks after birth with mouse PyMT tumor cells which expressed human MUC1. To this purpose tumor cells (1×10⁶/100 µl) were injected subcutaneously into the right flank of anesthetized mice. Tumor growth was determined every other day and palpable tumors were measured with a caliper (length × width).

Two weeks after tumor inoculation when an average tumor size of 20 mm² could be observed the mice were treated intraperitoneal (i.p.) with GGSK-1/30 ADCs (200 µg/100 µl) and tumor size was monitored every other day.

## Claims

1. An antibody drug conjugate comprising an antibody that specifically binds to tumor-associated Mucin 1 (TA-MUC1) of human epithelial tumor cells, but not to MUC1 on highly glycosylated healthy epithelial cells, **characterized in that** the antibody is coupled to a cytotoxic agent selected from the group consisting of a tubulin inhibitor or a topoisomerase inhibitor.

2. The antibody drug conjugate according to claim 1, wherein the tubulin inhibitor is emtansine (DM1), or the topoisomerase inhibitor is deruxtecan (DX) or SN-38.

3. The antibody drug conjugate according to claim 2, wherein the tubulin inhibitor is emtansine (DM1) linked via succinimidyl-trans-4-(maleimidylmethyl) cyclohexane-1-carboxylate (SMCC) to a -NH₂ of lysine residue of the antibody.

4. The antibody drug conjugate according to claim 3, wherein the cytotoxic agent coupled to the antibody via the lysine residue is SMCC-DM1 comprising the formula:

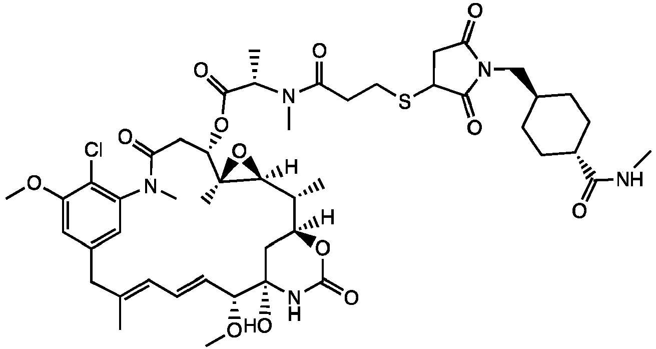

5. The antibody drug conjugate according to claim 2, wherein the topoisomerase inhibitor is deruxtecan (DX) linked via maleimidocaproyl (MC) which is covalently connected via a Gly Gly Phe Gly peptidyl spacer (GGFG) to a -SH of cysteine residue of the antibody.

6. The antibody drug conjugate according to claim 5, wherein the cytotoxic agent coupled to the antibody via the cysteine residue is MC-GGFG-DX comprising the 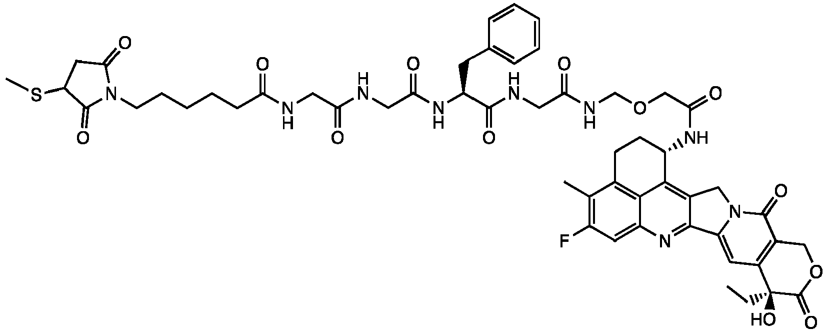 formula:

7. The antibody drug conjugate according to claim 2, wherein the topoisomerase inhibitor is SN-38 linked via maleimidocaproyl (MC) to a -SH of cysteine residue of the antibody.

8. The antibody drug conjugate according to claim 6, wherein the cytotoxic agent coupled to the antibody via the cysteine residue is MC-SN-38 comprising the formula:

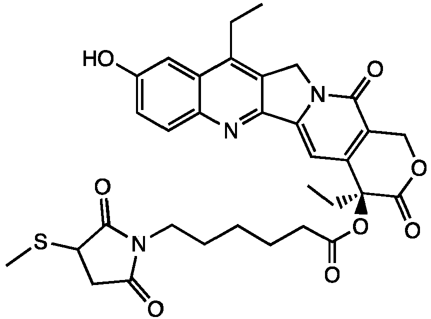

9. The antibody drug conjugate according to any one of claims 1 to 8, wherein the antibody is a monoclonal antibody directed against TA-MUC1.

10. The antibody drug conjugate according to claim 1, wherein the monoclonal antibody is GGSK-1/30.

11. The antibody drug conjugate according to any one of claims 1 to 10, wherein the antibody comprises the TA-MUC1 binding sites of the amino acid sequences SEQ ID NO: 1 to SEQ ID NO: 6.

12. The antibody drug conjugate according to claim 9, wherein the antibody comprises an amino acid sequence of any one of SEQ ID NO: 7 to 9.

13. An antibody drug conjugate according to any one of claims 1 to 12 for use in the treatment of malignant epithelial tumors.

14. The antibody drug conjugate for the use according to claim 13, wherein the malignant epithelial tumor is selected from the group consisting of breast cancer, pancreatic cancer, lung cancer, colon cancer, prostate cancer, bladder cancer, squamous cell carcinoma, endometrial adenocarcinoma.

15. A pharmaceutical composition, comprising an antibody drug conjugate according to any one of claims 1 to 12.
